# EUROPEAN PATENT APPLICATION

(11) **EP 4 555 959 A1**
(43) Date of publication of application: **21.05.2025**
(21) Application number: 23210107.1
(22) Date of filing: 15.11.2023
(51) Int. Cl.: A61B 34/10

(54) **TECHNIQUE ENABLING DETERMINATION OF A PLANNED POSE OF A MEDICAL IMPLANT**

(71) Applicant: Stryker European Operations Limited, Carrigtwohill, Co. Cork T45 HX08 (IE)
(72) Inventor: KHORWAL, Renu, 110063 New Delhi (IN); RAINA, Ravi Kiran, 201301 Noida (IN)
(74) Representative: Schott, Jakob Valentin

(57) **Abstract**

Methods enabling determination of a planned pose of a medical implant based on medical image data are disclosed. Constraint information is provided by a server to a surgical planning station, the constraint information indicating at least one constraint to be used by the surgical planning station for determining a planned pose of a medical implant. The planning station obtains medical image data of at least a portion of a patient's body in which portion the medical implant is to be implanted, and determines a planned pose of the medical implant based on the at least one constraint and the medical image data. An apparatus, a computer program and a system are also provided.

## Description

### TECHNICAL FIELD

The present disclosure provides a method enabling determination of a planned pose of a medical implant based on medical image data. An apparatus, a computer program and a system are also provided.

### BACKGROUND

In various surgical scenarios, poses of medical implants to be implanted in a patient's body are planned before conducting the surgical procedure. Such planning may be conducted manually by a surgeon based on pre-operative medical image data of the patient's body. For example, a computed tomography, CT, scan of the patient's spine may be acquired and the surgeon may be provided with two-dimensional digitally rendered radiographs, DRRs, generated based on the CT scan. The surgeon may then define positions in these DRRs the plan poses of pedicle screws that are to be implanted into the patient's vertebrae later on. Of course, such pre-planning of poses may also be used to plan poses of non-spinal implants.

Some solutions support the surgeon in the pose planning phase by providing a proposal for a planned pose of a given medical implant. For example, a pose-planning algorithm can be locally deployed on a planning station and determine a planned pose based on the medical image data of the patient and predefined constraint information. Referring to the spinal example mentioned above, a pose-planning algorithm deployed on a planning station may determine a planned pose of a pedicle screw based on the DRRs, using predefined geometrical properties of the pedicle screw such as a predefined diameter and predefined length thereof.

In current solutions, the accuracy of the planned poses will remain the same, irrespective of the number of planned poses that were determined by the pose-planning algorithm over time.

### SUMMARY

There is a need for a technique that solves one or more of the above or other problems.

According to a first aspect, a method enabling determination of a planned pose of a medical implant based on medical image data is provided. The method is performed by a surgical planning station. The method comprises receiving constraint information from a server, the constraint information indicating at least one constraint to be used by the surgical planning station for determining a planned pose of a medical implant. The method further comprises obtaining medical image data of at least a portion of a patient's body in which portion the medical implant is to be implanted. The method further comprises determining a planned pose of the medical implant based on the at least one constraint and the medical image data.

According to a second aspect, a method enabling determination of a planned pose of a medical implant based on medical image data is provided. The method is performed by a server. The method comprises transmitting constraint information to a surgical planning station, the constraint information indicating at least one constraint to be used by the surgical planning station for determining a planned pose of a medical implant based on medical image data of at least a portion of a patient's body in which the medical implant is to be implanted.

The following applies to both the method according to the first aspect and the method according to the second aspect.

The constraint information may indicate only the at least one constraint. The constraint information may be provided from the server to the planning station as part of a dataset consisting of non-executable data. The dataset may not comprise executable computer program code. The method(s) may not comprise providing a computer program from the server to the planning station.

The constraint information and/or the at least one constraint may be associated with a type of a medical implant (e.g., defined by user input obtained by the planning station and/or the server). The at least one constraint may define a type of the medical implant.

The at least one constraint may define one or more geometrical properties of the medical implant. The one or more geometrical properties may include a size of the medical implant, a shape of the medical implant, a volume of the medical implant, and/or an outline of the medical implant. For example, the medical implant is a pedicle screw. In this case, the at least one constraint may define a diameter and/or a length of the pedicle screw.

Alternatively, or in addition, the at least one constraint may define one or more spatial relationships between the medical implant and an anatomical element of a (e.g., the) patient's body. The one or more spatial relationships may include a predefined distance between at least a portion of the medical implant and the anatomical element, a predefined position of at least a portion of the medical implant relative to the anatomical element, and/or a predefined orientation of at least a portion of the medical implant relative to the anatomical element. For example, the medical implant is a pedicle screw and the anatomical element is a vertebra. In this case, the at least one constraint may define a pose of a screw insertion trajectory relative to the vertebra.

The planning station may be equipped with a surgical planning program. Determining the planned pose may comprise processing, by the surgical planning program, the at least one constraint and the medical image data.

The medical image data may be hidden from the server, at least until the planned pose has been determined. Alternatively, or in addition, (e.g., any) information derived from the medical image data may be hidden from the server, at least until the planned pose has been determined.

The at least one constraint may be provided from the server to the surgical planning station pre-operatively. The step of receiving the constraint information of the method according to the first aspect may be performed pre-operatively. The step of transmitting the constraint information of the method according to the second aspect may be performed pre-operatively. Optionally, one or more subsequent steps of the method(s) may be performed intraoperatively. The method(s) may not comprise a surgical step. Each of the methods may be referred to as a computer-implemented method.

Feedback information may be provided from the surgical planning station to the server. The method according to the first aspect may comprise transmitting the feedback information to the server. The method according to the second aspect may comprise receiving the feedback information from the planning station. The feedback information indicates at least: (i) one or more previously determined planned poses, (ii) at least one constraint used in determining the one or more previously determined planned poses, (iii) one or more previously determined and user-adjusted planned poses, and/or (iv) at least one constraint used in determining the one or more previously determined and user-adjusted planned poses. The feedback information may be associated with one or more users (e.g., surgeons), one or more surgeries, one or more patients, one or more medical image types and/or one or more hospitals.

The at least one constraint to be used by the surgical planning station for determining the planned pose of the medical implant may be based on the feedback information. For example, the at least one constraint is or was determined by the server. The method according to the second aspect may comprise determining the at least one constraint, e.g. based on at least the feedback information.

For example, the at least one constraint is or was obtained as an output of a machine learning model trained using the feedback information as training data. The machine learning model may be installed on or accessed by (e.g., only) the server. The method according to the second aspect may comprise obtaining the at least one constraint from the machine learning model. The method according to the second aspect may comprise training the machine learning model based on the feedback information. The method according to the second aspect may comprise obtaining feedback information from a plurality of planning stations and training the machine learning model based thereon.

For example, the machine learning model is or was trained using federated learning. The method according to the first and/or second aspect may comprise training the machine learning model using federated learning (e.g., based on the feedback information of a plurality of planning stations).

The method according to the first aspect may comprise triggering a visualization of the determined planned pose, for example by overlaying an indication of the determined planned pose in a (e.g., two-dimensional or three-dimensional) rendering of the medical image data. Pose information indicative of the determined planned pose may be transmitted to the server, for example as (e.g., part of) the feedback information.

In one particular variant currently not reflected in the wording of the claims, the term "pose" as used herein is to be replaced with "pose and/or size and/or shape". In this case, instead or in addition to determining a planned pose of the medical implant, a planned size and/or a planned shape of the medical implant may be determined.

According to a third aspect, an apparatus is provided. The apparatus is configured to perform the method according to the first aspect. In this case, the apparatus may be configured as the planning station. Alternatively, the apparatus is configured to perform the method according to the second aspect. In this case, the apparatus may be configured as the server.

According to a fourth aspect, a computer program is provided. The computer program comprises instructions which, when performed by a processor, cause the processor to carry out the method according to the first aspect or according to the second aspect. The computer program may be carried by a carrier such as a data stream, a signal wave or a (e.g., non-transitory) computer-readable storage medium.

According to a fifth aspect, a system is provided. The system comprises a planning station configured to perform the method according to the first aspect. The system further comprises a server configured to perform the method according to the second aspect.

### SHORT DESCRIPTION OF THE FIGURES

Examples in accordance with the technique disclosed herein are explained below with reference to the figures, wherein:
- Fig. 1: illustrates an exemplary system in accordance with the present disclosure;
- Fig. 2: illustrates a flowchart of an exemplary method in accordance with the present disclosure; and
- Fig. 3: illustrates an exemplary automatic implant planning procedure in accordance with the present disclosure.

### DETAILED DESCRIPTION

Unless indicated otherwise, the reference signs used in the following denote the same or similar structural or functional features. In case an example shows more than one instance of a given entity, which entity is denoted with reference numeral "X", these instances may be referred to either as "X", or as "X-*n*" with *n* indicating the particular instance.

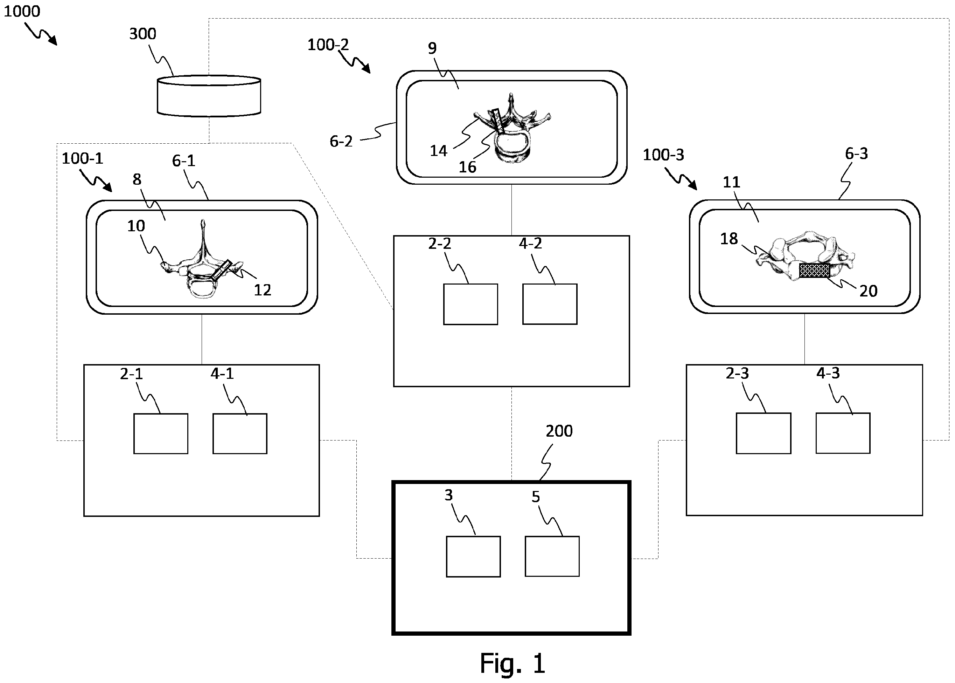

Fig. 1 illustrates an exemplary system 1000 in accordance with the present disclosure. The system 1000 comprises a plurality of planning stations 100-1 to 100-3, a server 200 communicatively connected to each of the planning stations 100-1 to 100-3, and a database 300 communicatively connected to one or more of the planning stations 100-1 to 100-3. Each planning station 100 comprises a processor 2 and a memory 4, the memory 4 storing instructions which, when executed by the processor 2 configure the planning system 100 to operate as disclosed herein. The server 200 also comprises a processor 3 and a memory 5, the memory 5 storing instructions which, when executed by the processor 3 configure the server 200 to operate as disclosed herein. Each of the planning systems 100-1 to 100-3 further comprises a display unit 6-1 to 6-3 configured to display a visualization. The database 300 may store medical image data of a patient's body. The database 300 may be part of a Picture Archiving and Communication System, PACS, for example a PACS of a hospital (e.g., in which the planning system 100 connected to said database 300 is also located). It is also possible for the database 300 to be connected to or part of a medical image acquisition device such as a magnetic resonance, MR, scanner or a computed tomography, CT, scanner, for example, which device may be configured to acquire the medical image data.

The sever 200 may be implemented as a single entity or as a service a cloud computing platform. The planning stations 100 on the other hand are each configured as local systems, for example stand-alone systems in one or more hospitals. The medical image data may be kept hidden from the server 200 for improved data protection. For example, the planning stations 100 may each be configured with a firewall that does not allow access to the medical image data by the server 200.

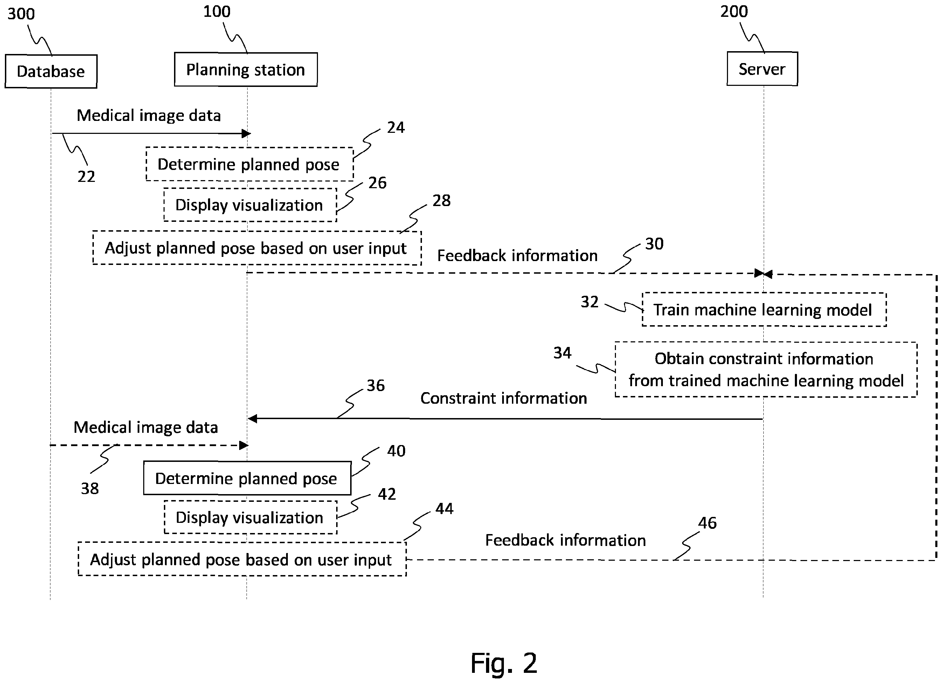

Fig. 2 illustrates a flowchart of an exemplary method in accordance with the present disclosure. The method may be performed by the system 1000 or components thereof and may comprise optional steps indicated with dashed lines. One or more or all of the steps (e.g., at least steps 32-36) may be performed pre-operatively.

In step 22, medical image data is obtained by the planning station 100 from the database 300. The medical image data comprises one or more medical images of at least a portion of a patient's body in which portion a medical implant is to be implanted. The medical image data may comprise two-dimensional and/or three-dimensional image data, for example a MR scan, a CT scan, an X-ray image or an ultrasonic image of the portion of the patient's body. Each planning station 100-1 to 100-3 may obtain different medical image data. For example, the planning station 100-1 may obtain medical image data of a first patient, the planning station 100-2 may obtain medical image data of a second patient and the planning station 100-3 may obtain medical image data of a third patient.

In optional step 24, the planning station 100 determines a planned pose for the implant to be implanted into the portion of the patient's body, based on the obtained medical image data. This step may be referred to as automatic implant planning. The planning station 100 may be equipped with a surgical planning program (e.g., stored on the memory 4) that processes at least the obtained medical image data to determine the planned pose.

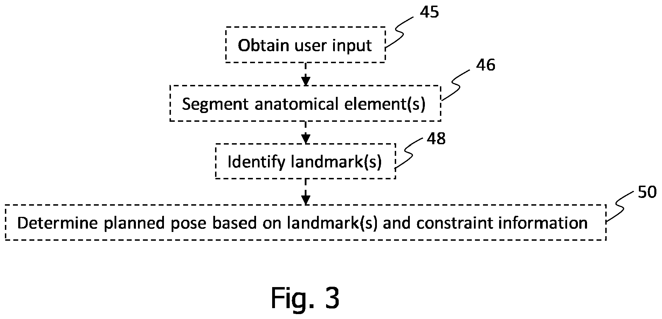

An exemplary automatic implant planning procedure that may be performed as part of step 24 (e.g., by the surgical planning program) is shown in Fig. 3 and comprises sub-steps 45, 46, 48, 50.

The planning station 100 may obtain a user input in sub-step 45, defining a type of the medical implant to be implanted (e.g., a bone screw, a bone plate, a spinal cage or the like) and, optionally, an anatomical element into or onto which the medical implant is to be implanted (e.g., a name of a vertebra, for example "L4").

In sub-step 46, at least an anatomical element into or onto which the medical implant is to be implanted is segmented based on the medical image data. Various image segmentation techniques that can be used in this context will be apparent to those skilled in the art.

In sub-step 48, one or more landmarks of the segmented anatomical element(s) are identified (e.g., by comparing the segmented shape with a predefined template having pre-marked landmarks).

In sub-step 50, a planned pose of the medical implant to be implanted is determined based on the segmented anatomical element(s) and/or based on the identified landmarks(s). In this sub-step, predefined constraint information is used for determining the planned pose. The predefined constraint information may be locally stored on the memory 4 (e.g., when installing the surgical planning program on the planning station 100). Alternatively, or in addition, the predefined constraint information may be (e.g., at least in part) defined by a user and/or obtained in sub-step 45. The predefined constraint information may indicate one or more predefined constraints to be used by the planning station 100 (e.g., the surgical planning program) for determining the planned pose, for example a predefined diameter and predefined length of a pedicle screw and a predefined angle between a screw insertion trajectory of the pedicle screw and a vertebral endplate of the vertebra into which the pedicle screw is to be implanted.

In optional step 26, a visualization is triggered to be displayed on the display device 6 of the planning station 100. The visualization at least indicates the planned pose determined in step 24. For example, a two-dimensional or three-dimensional rendering of one or more medical images comprised in the medical image data may be displayed with a virtual medical implant complying with the constraint information being overlaid onto the rendering in the determined planned pose. This is exemplarily illustrated in Fig. 1, where a visualization 8 is displayed on the display device 6-1, indicating a planned pose of pedicle screw 12 relative to vertebra 10 of the first patient, a visualization 9 is displayed on the display device 6-2, indicating a planned pose of pedicle screw 16 relative to vertebra 14 of the second patient, and a visualization 11 is displayed on the display device 6-3, indicating a planned pose of spinal cage 20 relative to vertebra 18 of the third patient. Other variants of indicating the planned pose are also possible, for example by displaying a screw trajectory, a screw outline and/or positions of one or more predefined points of the medical implant (e.g., a screw tip).

The planned pose determined in step 24 may not comply with a user's preference. Thus, the method may allow a user to change the determined pose and/or the medical implant which pose was determined by providing suitable user input. Accordingly, in optional step 28, a user of the planning station 100 provides a user input to the planning station 100, causing the planning station 100 (e.g., the surgical planning program) to adjust the planned pose that was determined in step 24 accordingly based on the user input. This may, in turn, result in an adjustment of the at least one constraint that was used for determining the planned pose.

In optional step 30, the planning station 100 transmits feedback information to the server 200. The feedback information may be indicative of one or more of (i) the planned pose, (ii) the predefined constraint information used for determining the planned pose in step 24, (iii) the user input, and (iv) the adjusted at least one constraint resulting from the adjusted planned pose. Alternatively, or in addition, the feedback information transmitted in step 30 may indicate (i) one or more previously determined planned poses (e.g., as determined in step 24), (ii) at least one (e.g., predefined) constraint used in determining the one or more previously determined planned poses, (iii) one or more previously determined and user-adjusted planned poses (e.g., as adjusted in step 28), and/or (iv) at least one (e.g., predefined) constraint used in determining the one or more previously determined and user-adjusted planned poses. The feedback information may not comprise the medical image data previously obtained by the planning station 100.

The server 200 may use the feedback information obtained from one or more planning stations 100 for one or more surgeries and/or one or more patients and determine (e.g., updated and/or improved) constraint information based thereon. As a mere example, if the predefined constraint information indicates a preferred angle of 70° between a screw trajectory and a vertebral endplate and the feedback information obtained from various planning stations 10 indicates a preferred angle of on average 60°, it can be assumed that the predefined constraint information does not conform with the preferences of the users of the planning stations 10. Thus, the constraint information may be updated by the server. To this end, the server 200 may train a machine learning model based on the feedback information in optional step 32. The machine learning model may run on the server 200 or be accessed by the server 200 (e.g., in case it is provided by a cloud computing platform). The server 200 may input at least the feedback information as training data into the machine learning model to train the same.

It is also possible for the server to employ federated learning to train the machine learning model. In this case, separate instances of the machine learning model may be individually trained with feedback information obtained from multiple planning stations 100 such that each instance is associated with a different planning station 100. It is also possible to associate the different instances to different patients and/or different surgeries and/or different hospitals. The trained instances can then be aggregated by the server 200 to, in result, obtain a single trained machine learning model.

The trained machine learning model may be configured to provide, as an output, (e.g., updated and/or improved) constraint information. Accordingly, in optional step 34, the server 200 obtains such constraint information from the trained machine learning model. The updated and/or improved constraint information can then be supplied to the planning station(s) 100 to improve future planning outcomes.

In step 36, the server 200 transmits (e.g., the) constraint information to the planning station 100. The constraint information transmitted in step 36 may have been determined by the server 200, for example using the trained machine learning model according to step 34. The constraint information transmitted in step 36 may differ from the predefined constraint information used in step 24. The constraint information transmitted in step 36 indicates at least one constraint to be used by the surgical planning station 100 (e.g., the surgical planning program) for determining a planned pose of a medical implant based on medical image data of at least a portion of a patient's body in which the medical implant is to be implanted. In case the machine learning model was trained (e.g., in step 32) using feedback information (e.g., as obtained in step 30), one may say that the at least one constraint indicated by the constraint information is based on said feedback information.

The at least one constraint indicated by the constraint information transmitted in step 36 may define one or more geometrical properties of the medical implant. For example, the one or more geometrical properties may include a size of the medical implant, a shape of the medical implant, a volume of the medical implant, and/or an outline of the medical implant. If the medical implant is a pedicle screw, the at least one constraint may define a diameter and/or a length of the pedicle screw.

Alternatively, or in addition, the at least one constraint indicated by the constraint information transmitted in step 36 may define one or more spatial relationships between the medical implant and an anatomical element of a patient's body (e.g., into which or onto which the implant is to be placed). For example, the one or more spatial relationships include a predefined distance between at least a portion of the medical implant and the anatomical element, a predefined position of at least a portion of the medical implant relative to the anatomical element, and/or a predefined orientation of at least a portion of the medical implant relative to the anatomical element. If the medical implant is a pedicle screw and the anatomical element is a vertebra, the at least one constraint may define a pose of a screw insertion trajectory relative to the vertebra.

In optional step 38, medical image data of at least a portion of a patient's body, in which portion the medical implant is to be implanted, is obtained. It is also possible to avoid step 38 and instead use the same medical image data already obtained in step 22, if desired. The medical image data that can be obtained in optional step 38 may be associated with a different patient, surgery, anatomical region and/or image acquisition timepoint than the medical image data obtained in step 22.

In step 40, a planned pose of the medical implant is determined based on the medical image data and further based on the (e.g., updated and/or improved) constraint information received in step 36. This differs from the determination of step 24 in that rather than using predefined constraint information, the constraint information provided by the server 200 in step 36 is used. One may say that the determination of the planned pose in step 40 is influenced by the server 200, as it relies on the (e.g., updated and/or improved) constraint information previously provided by the server 200. This gives an operator of the server (e.g., a manufacturer of the planning station 100) the opportunity to improve the planning by the planning stations 100 without having to roll out new versions of the surgical planning program and installing the same on each planning station 100. Compared with a complete surgical planning program comprising executable computer program portions, the constraint information requires less storage space and can be transmitted using lower transmission resources.

In optional step 42, a visualization is triggered to be displayed on the display unit 6. This visualization may be configured similar as the visualization described with reference to optional step 26, but be based on the planned pose determined in step 40 instead of the planned pose determined in step 24 (and, optionally, be based on medical image data obtained in step 38 instead of 22). Step 40 may comprise one or more of the sub-steps 45-50, using the constraint information obtained in step 36 in sub-step 50 and, optionally, the medical image data obtained in step 38 in sub-step 46 and/or 48.

As described above for optional step 28, the user may not approve the planned pose determined in step 40 and provide corresponding user input to initiate an adjustment of the planned pose in step 44.

In optional step 46, the planning station 100 transmits feedback information to the server 200. The feedback information may be indicative of one or more of (i) the planned pose determined in step 40, (ii) the (e.g., updated and/or improved) constraint information used for determining the planned pose in step 40, (iii) the user input initiating the adjustment of the planned pose, and (iv) the adjusted at least one constraint resulting from the adjusted planned pose. Alternatively, or in addition, the feedback information transmitted in step 46 may indicate (i) one or more previously determined planned poses (e.g., as determined in steps 24 and/or 40), (ii) at least one (e.g., updated and/or improved) constraint used in determining the one or more previously determined planned poses, (iii) one or more previously determined and user-adjusted planned poses (e.g., as adjusted in step 44), and/or (iv) at least one (e.g., updated and/or improved) constraint used in determining the one or more previously determined and user-adjusted planned poses. Also here, the feedback information may not comprise the medical image data previously obtained by the planning station 100.

The method may then continue with the server 200 determining or obtaining updated and/or improved constraint information based on the feedback obtained in step 46 and optionally further based on the feedback obtained in step 30. In this manner, user adjustments of planned poses determined based on the server-provided constraint information can be fed back to the server to improve the constraint information over and over again, thereby resulting in planned poses that better comply with the user's requirements.

The technique disclosed herein will now be rephrased in other words to explain the relevant teachings in more detail.

Generally speaking, planning of poses of medical implants and/or dimensions thereof is a time-consuming but important step in (e.g., spinal) surgical navigation procedures. This functionality may be particularly advantageous in robot-assisted surgeries, as the intended poses may be required to be communicated to a surgical robot.

Automatic planning of implants may reduce the overall procedure time and provide greater accuracy. Such automatic planning procedures may be based on the detection of structural and anatomical landmarks in medical images (e.g., in steps 46 and 48). Machine learning may be used for segmenting anatomical elements and/or identifying prominent landmarks (e.g., in steps 46 and/or 48) such as a pedicle, vertebra body, spinous process and transverse processes). With the identified landmarks, a surgical planning program determine a planned pose of a desired implant.

In some solutions, a fixed statistical model is used for automatic implant planning, which relies on predefined constraint information. Such a solution is not perfect and surgeons might need to modify the planned pose to accommodate for anatomic variations, previous surgeries, or some underlying vertebra anomaly. Hence, also to provide a better plan and improve overall planning accuracy, the technique disclosed herein allows updating the statistical model for automatic implant planning, in particular the predefined constraint information, based on feedback information (e.g., history data of adjustments made by surgeons in determined implant poses).

One may say that the present technique can improve automatic implant planning based on feedback data logged and saved on the server 200 (e.g., part of a cloud computing platform) without need to update the surgical planning program installed on the planning stations 1000. This may improve the accuracy of the determined poses without the need to redeploy the surgical planning program. The server 200 may calculate an improved weightage of constraints to be used for determining a planned pose of an implant, for example using statistical and/or machine learning methods. The improved weights may be fed as part of the updated constraint information in step 36. This in turn may improve the surgical planning program such that subsequently planned poses provide higher clinical acceptance.

Put in other words, one concept of the technique disclosed herein lies in dynamically adapting the constraint(s) used for determining a planned pose of an implant based on feedback information collected for adjustments made to previously determined planned poses. This may provide for better constraint(s) for automatic implant pose planning (e.g., vertebrae specific parameters like pedicle transverse angle, length and diameter) based on changes made by a surgeon to a previously determined planned pose (e.g., of a pedicle screw). Surgeon can make changes to the determined planned pose to adapt to anatomic variations, previous surgeries, or some underlying vertebra anomaly. Based on these changes optimized weights can be calculated by the server as part of the updated constraint information, using statistical and/or machine learning, and these optimized algorithm weights can be fed to the existing algorithm (e.g., the surgical planning program) to achieve a greater level of accuracy hence forth expediting the planning procedure.

The technique disclosed herein is not limited to screw plan optimization and spinal applications. It can be applied to other anatomical regions (e.g., shoulder, hip or cranial) and may be used to plan poses of implants. Also in such cases, improved planned poses of implants and updated constraint information can be provided without the need change the source code of the surgical planning program. That is, the surgical planning program may use the (e.g., updated and/or improved) constraint information as a parameter input for determining the planned pose in step 40. Further modifications and advantages of the technique disclosed herein will be apparent to those skilled in the art.

## Claims

1. A method enabling determination of a planned pose of a medical implant (12; 16; 20) based on medical image data, the method being performed by a surgical planning station (100) and comprising:
receiving (36) constraint information from a server (200), the constraint information indicating at least one constraint to be used by the surgical planning station (100) for determining a planned pose of a medical implant (12; 16; 20);
obtaining (38) medical image data of at least a portion of a patient's body in which portion the medical implant (12; 16; 20) is to be implanted; and
determining (40) a planned pose of the medical implant (12; 16; 20) based on the at least one constraint and the medical image data.

2. A method enabling determination of a planned pose of a medical implant (12; 16; 20) based on medical image data, the method being performed by a server (200) and comprising:
transmitting (36) constraint information to a surgical planning station (100), the constraint information indicating at least one constraint to be used by the surgical planning station (100) for determining a planned pose of a medical implant based on medical image data of at least a portion of a patient's body in which the medical implant (12; 16; 20) is to be implanted.

3. The method of claim 1 or 2, wherein
the at least one constraint defines one or more geometrical properties of the medical implant (12; 16; 20).

4. The method of claim 3, wherein
the one or more geometrical properties include a size of the medical implant (12; 16; 20), a shape of the medical implant (12; 16; 20), a volume of the medical implant (12; 16; 20), and/or an outline of the medical implant (12; 16; 20).

5. The method of claim 3 or 4, wherein
the medical implant (12; 16) is a pedicle screw and the at least one constraint defines a diameter and/or a length of the pedicle screw.

6. The method of any one of claims 1 to 5, wherein
the at least one constraint defines one or more spatial relationships between the medical implant (12; 16; 20) and an anatomical element (10; 14; 18) of a patient's body.

7. The method of claim 6, wherein
the one or more spatial relationships include a predefined distance between at least a portion of the medical implant (12; 16; 20) and the anatomical element (10; 14; 18), a predefined position of at least a portion of the medical implant (12; 16; 20) relative to the anatomical element (10; 14; 18), and/or a predefined orientation of at least a portion of the medical implant (12; 16; 20) relative to the anatomical element (10; 14; 18).

8. The method of claim 6 or 7, wherein
the medical implant (12; 16) is a pedicle screw and the anatomical element (10; 14; 18) is a vertebra, and wherein the at least one constraint defines a pose of a screw insertion trajectory relative to the vertebra.

9. The method of any one of claims 1 to 8, wherein
the planning station (100) is equipped with a surgical planning program, wherein determining the planned pose comprises processing, by the surgical planning program, the at least one constraint and the medical image data.

10. The method of any one of claims 1 to 9, wherein
the medical image data and optionally any information derived from the medical image data is hidden from the server (200), at least until the planned pose has been determined.

11. The method of any one of claims 1 to 10, wherein
the at least one constraint is provided from the server (200) to the surgical planning station (100) pre-operatively, wherein optionally one or more subsequent steps of the method are performed intraoperatively.

12. The method of any one of claims 1 to 11, wherein
feedback information is provided (30; 46) from the surgical planning station to the server, the feedback information indicating at least: (i) one or more previously determined planned poses, (ii) at least one constraint used in determining the one or more previously determined planned poses, (iii) one or more previously determined and user-adjusted planned poses, and/or (iv) at least one constraint used in determining the one or more previously determined and user-adjusted planned poses,
wherein the at least one constraint to be used by the surgical planning station (100) for determining the planned pose of the medical implant (12; 16; 20) is based on the feedback information.

13. The method of any one of claims 1 to 12, wherein
the at least one constraint is or was determined by the server (200).

14. The method of claims 12 and 13, wherein
the at least one constraint is or was obtained as an output of a machine learning model trained using the feedback information as training data.

15. The method of claim 14, wherein
the machine learning model is or was trained using federated learning.

16. An apparatus (100; 200) configured to perform the method steps of the method according to any one of claims 1 to 15.

17. A computer program comprising instructions which, when performed by a processor (2; 3), cause the processor (2; 3) to carry out the method according to any one of claims 1 to 15, the computer program being optionally carried by a carrier (4; 5).

18. A system (1000) comprising:
a planning station (100) configured to perform the method of at least claim 1; and
a server (200) configured to perform the method of at least claim 2.

## Amended claims

### Amended claims in accordance with Rule 137(2) EPC.

1. A method enabling determination of a planned pose of a medical implant (12; 16; 20) based on medical image data, the method being performed by a surgical planning station (100) and comprising:
receiving (36) constraint information from a server (200), the constraint information indicating at least one constraint to be used by the surgical planning station (100) for determining a planned pose of a medical implant (12; 16; 20);
obtaining (38) medical image data of at least a portion of a patient's body in which portion the medical implant (12; 16; 20) is to be implanted; and
determining (40) a planned pose of the medical implant (12; 16; 20) based on the at least one constraint and the medical image data; wherein
the medical image data is hidden from the server (200), at least until the planned pose has been determined.

2. A method enabling determination of a planned pose of a medical implant (12; 16; 20) based on medical image data, the method being performed by a server (200) and comprising:
transmitting (36) constraint information to a surgical planning station (100), the constraint information indicating at least one constraint to be used by the surgical planning station (100) for determining a planned pose of a medical implant based on medical image data of at least a portion of a patient's body in which the medical implant (12; 16; 20) is to be implanted; wherein
the medical image data is hidden from the server (200), at least until the planned pose has been determined.

3. The method of claim 1 or 2, wherein
the at least one constraint defines one or more geometrical properties of the medical implant (12; 16; 20).

4. The method of claim 3, wherein
the one or more geometrical properties include a size of the medical implant (12; 16; 20), a shape of the medical implant (12; 16; 20), a volume of the medical implant (12; 16; 20), and/or an outline of the medical implant (12; 16; 20).

5. The method of claim 3 or 4, wherein
the medical implant (12; 16) is a pedicle screw and the at least one constraint defines a diameter and/or a length of the pedicle screw.

6. The method of any one of claims 1 to 5, wherein
the at least one constraint defines one or more spatial relationships between the medical implant (12; 16; 20) and an anatomical element (10; 14; 18) of a patient's body.

7. The method of claim 6, wherein
the one or more spatial relationships include a predefined distance between at least a portion of the medical implant (12; 16; 20) and the anatomical element (10; 14; 18), a predefined position of at least a portion of the medical implant (12; 16; 20) relative to the anatomical element (10; 14; 18), and/or a predefined orientation of at least a portion of the medical implant (12; 16; 20) relative to the anatomical element (10; 14; 18).

8. The method of claim 6 or 7, wherein
the medical implant (12; 16) is a pedicle screw and the anatomical element (10; 14; 18) is a vertebra, and wherein the at least one constraint defines a pose of a screw insertion trajectory relative to the vertebra.

9. The method of any one of claims 1 to 8, wherein
the planning station (100) is equipped with a surgical planning program, wherein determining the planned pose comprises processing, by the surgical planning program, the at least one constraint and the medical image data.

10. The method of any one of claims 1 to 9, wherein
any information derived from the medical image data is hidden from the server (200), at least until the planned pose has been determined.

11. The method of any one of claims 1 to 10, wherein
the at least one constraint is provided from the server (200) to the surgical planning station (100) pre-operatively, wherein optionally one or more subsequent steps of the method are performed intraoperatively.

12. The method of any one of claims 1 to 11, wherein
feedback information is provided (30; 46) from the surgical planning station to the server, the feedback information indicating at least: (i) one or more previously determined planned poses, (ii) at least one constraint used in determining the one or more previously determined planned poses, (iii) one or more previously determined and user-adjusted planned poses, and/or (iv) at least one constraint used in determining the one or more previously determined and user-adjusted planned poses,
wherein the at least one constraint to be used by the surgical planning station (100) for determining the planned pose of the medical implant (12; 16; 20) is based on the feedback information.

13. The method of any one of claims 1 to 12, wherein
the at least one constraint is or was determined by the server (200).

14. The method of claims 12 and 13, wherein
the at least one constraint is or was obtained as an output of a machine learning model trained using the feedback information as training data.

15. The method of claim 14, wherein
the machine learning model is or was trained using federated learning.

16. An apparatus (100; 200) configured to perform the method steps of the method according to any one of claims 1 to 15.

17. A computer program comprising instructions which, when performed by a processor (2; 3), cause the processor (2; 3) to carry out the method according to any one of claims 1 to 15, the computer program being optionally carried by a carrier (4; 5).

18. A system (1000) comprising:
a planning station (100) configured to perform the method of at least claim 1; and
a server (200) configured to perform the method of at least claim 2.
